# EUROPEAN PATENT SPECIFICATION

(11) **EP 1 152 779 B1**
(45) Date of publication and mention of the grant of the patent: **22.11.2006**
(21) Application number: 00901324.4
(22) Date of filing: 11.01.2000
(51) Int. Cl.: A61M 1/00, A61M 27/00

(54) **DEVICE FOR DRAINING EXUDATIVE MATERIAL FROM AN ENDOCORPOREAL CAVITY OF A PATIENT**
VORRICHTUNG ZUM DRAINIEREN VON ABGESONDERTEM MATERIAL AUS EINER KÖRPERHÖHLE EINES PATIENTEN
DISPOSITIF POUR DRAINER UNE SUBSTANCE EXSUDATIVE D'UNE CAVITE INTRACORPORELLE D'UN PATIENT

(30) Priority: 12.01.1999 IT MI990032
(43) Date of publication of application: 14.11.2001
(73) Proprietor: Tricarico, Michelina, 20098 San Giuliano Milanese (IT)
(72) Inventor: Tricarico, Michelina, 20098 San Giuliano Milanese (IT)
(74) Representative: Bottero, Claudio
(86) International application number: PCT/IT2000/000009
(87) International publication number: WO 2000/041743

(56) References cited:
- EP-A- 0 608 846
- EP-A- 0 864 333
- WO-A-97/36632
- DE-A- 3 933 088
- US-A- 3 642 004
- US-A- 3 777 757
- US-A- 3 805 794
- US-A- 4 676 782

## Description

### Field of the invention

In one general aspect thereof, the present invention relates to a device for draining exudative material from an endocorporeal cavity of a patient.

More particularly, the present invention relates to a draining device comprising:
a) a draining tube having opposite inlet and discharge openings for the exudative material;
b) means for fastening said draining tube to the patient's skin, comprising a deformable sleeve, externally extending around the draining tube to which the same is sealingly fixed, and with which the sleeve defines an expandable interspace;
c) means for feeding a sterile expansion fluid to at least one end portion of said expandable interspace, said means including at least one duct at least partly integrally formed in the draining tube, having an inlet opening accessible from the outside of the draining tube and at least one outlet opening selectively in fluid communication with said at least one end portion of the expandable interspace.

In this description and in the following claims, the expression "exudative material" is used to indicate any liquid serous, haematic or purulent exudate produced by an inflammatory or irritative process, such as for example serum, blood, colliquated necrotic material, purulent material in general (pus).

On the other hand, the expression "endocorporeal cavity" is used to indicate any subcutaneous cavity, whether natural, neo-formed or consequent to surgery, wherein exudative material may be collected.

### Prior Art

As is known, one of the needs to be met in the clinical practice and, in particular, in the postoperative course, during which it is necessary to drain the exudative material collected in an endocorporeal cavity, whether natural or formed as a consequence of surgery, is that of stably fastening the draining device to the patient's skin.

To this end, draining devices are known such as those disclosed by Italian patent no. 1.269.980 which are provided with means for fastening the draining tube to the patient's skin, essentially constituted by a deformable sleeve, externally extending around the draining tube to which the same is sealingly fixed, and with which the sleeve defines an expandable interspace.

Additional devices of this kind are also disclosed by US patents Nos. 3,777,757 and 3,805,794 which illustrate inflatable bag catheters provided with a pliable elongated bag disposed generally at the distal end thereof. Both these devices are also provided with check valves positioned along an inflating tube or at an end of a connector arm in fluid communication with the pliable elongated bag.

Said devices are fastened to the patient's skin by expanding the aforementioned interspace with sterile fluid fed in a duct at least partly formed in the draining tube, which duct is in fluid communication with the end portions of the expandable interspace.

Although such devices allow to fasten the draining tube to the patient's skin without having to resort to suture, the disadvantages of which have long been known and are especially felt in the case of long postoperative courses, they cannot prevent accidental passages of the expansion fluid between the end portions of the expandable interspace, which are in reciprocal communication through the feeding duct of the expansion fluid.

The occurrence of said accidental passages, however, could compromise the fastening stability of the device, with consequent troubles for the patient and a possible arise of infections due, for example, to the penetration of non-sterile portions of the draining tube into the endocorporeal cavity, or to the extraction of the tube itself when a possible passage of the expansion fluid causes a diameter reduction of the deformable sleeve.

Finally, it is also known in the art - as disclosed by European patent application EP 0 608 794 - a unidirectional surgical drain for the removal from the body of blood, secretions and other fluids or semifluids comprising unidirectional inlets along the tube's wall and a unidirectional valve or a plug at the distal opening of the drain tube, enabling the body fluids to flow only in one direction from the outside into the inside of the tube.

### Summary of the invention

Accordingly, the technical problem underlying the present invention is that of providing a device for draining exudative material from an endocorporeal cavity of a patient which may be firmly and durably fastened to the patient's skin, and which is free from the disadvantages mentioned with reference to the cited prior art.

This technical problem is solved thanks by a draining device as disclosed in claim 1 attached herewith.

According to the invention, the check-valve means provided in said at least one outlet opening allows to introduce the sterile expansion fluid into the expandable interspace during the fastening step of the draining tube to the patient's skin, while cutting off the fluid communication between the feeding duct and the expandable interspace at the end of the feeding step, thus preventing any accidental passage of the sterile expansion fluid out of the expanded portion of the interspace.

More particularly, the valve means allows a unidirectional flow of fluid towards the interspace in the presence of an overpressure, but they close the outlet opening of the fluid at the end of the feeding overpressure.

According to the invention, said at least one outlet opening is defined by at least one through notch, substantially slant shaped, radially formed in a wall of the draining tube, and the check-valve means is preferably essentially constituted by elastically deformable matching adjoining portions of the wall of the draining tube defined by said notch.

Still more preferably, said at least one outlet opening is defined by a couple of cross-notches, substantially perpendicular with one another.

In this embodiment, the elastically deformable adjoining portions of the wall of the draining tube which are usually in contact with one another get deformed at the notch which defines the same as a consequence of the pressure exerted by the sterile expansion fluid during the feeding step, thus allowing the fluid communication between the feeding duct and the expandable interspace. Once the sterile fluid feeding has come to an end, said portions return again to be in contact with one another so as to prevent the outflow of the sterile fluid, thus firmly keeping the device in position.

In an embodiment of the invention, the deformable sleeve comprises two deformable portions which are structurally independent from one another, and which define respective expandable interspaces with the draining tube.

In this way, the fastening stability to the patient's skin may be further increased.

According to a further embodiment, the means for feeding the sterile fluid comprises two feeding ducts associated to the draining tube at diametrically opposite sides thereof, each of the aforementioned feeding ducts being selectively in fluid communication with one of said expandable interspaces.

In this way, the expansion of the two structurally independent portions of the expandable interspace occurs by selectively feeding the sterile fluid in the two ducts. Advantageously, this feature allows to obtain the desired expansion in one of the portions of the expandable interspace independently of the expansion carried out in the other portion.

Preferably, the deformable sleeve coaxially extends around the draining tube along a portion thereof having a predetermined length, thus reaching optimal characteristics of stability of the device under all conditions of use.

In a preferred embodiment, the duct for feeding the sterile fluid is provided with a reinforcing tubular element, mounted close to its inlet opening. Advantageously, the presence of the reinforcing tubular element allows to prevent accidental perforations of the walls of the draining tube while feeding the sterile expansion fluid, which is generally done with a conventional syringe.

For the purposes of the invention, the reinforcing tubular element may be made either of metal or of plastics, provided that the selected material is compatible with the material of the draining tube.

Preferably, the duct for feeding the sterile fluid is sealed by suitable means, such as for example a self-sealing pierceable pad fastened near the inlet opening of the feeding duct, or a suitably shaped removable plug housed in said inlet opening.

Advantageously, these sealing closing means has the function of preventing accidental leaks of the residual sterile expansion fluid present in the feeding duct.

Preferably, the self-sealing pierceable pad is mounted into the feeding duct upstream of the reinforcing element.

Still more preferably, the pad is mounted in the feeding duct between two reinforcing elements. In this way, a possible accidental shifting of the pad along the duct itself by the syringe used for feeding the sterile fluid is prevented while feeding the sterile fluid.

According to the invention, the duct for feeding the sterile fluid is at least partly integral with the draining tube.

Preferably, the feeding duct comprises an initial portion which is externally fixed to the draining tube by means of a resectable tongue. In this way, the feeding duct may be structurally independent of the draining tube at said initial portion, thus facilitating the feeding operations of the sterile fluid into the feeding duct. In particular, should the need arise of feeding further portions of the sterile fluid into the expandable interspace while using the device, the feeding of sterile fluid may be advantageously carried out without removing the discharge opening of the draining tube from the collecting bag of the drained exudative material.

In an embodiment of the invention, at least one of the end portions of the sleeve may have a different elasticity and thus, it may be made of a material having different elastic properties with respect to the remaining portions of the sleeve itself. Such embodiment may be advantageous in draining exudative materials from cavities located in parts of the body in which the skin is very delicate, and for which it is necessary a mainly radial expansion of the interspace instead of a longitudinal one.

In a further embodiment of the invention, the draining tube may comprise a flattened intracutaneous portion provided with a plurality of openings for sucking the exudative material. Such embodiment is especially advantageous in those cases in which the endocorporeal cavity occupies a portion which mainly extends in longitudinal direction.

In an additional embodiment, furthermore, the draining tube may have a substantially elliptical cross-section so as to facilitate, once removed, the cicatrization of the opening made in the skin to house it.

Preferably, the draining device further comprises a mark formed at a predetermined position on the draining tube for indicating the correct positioning of the device through the skin of the patient.

Additional features and advantages of the present invention will be more readily apparent from the following detailed description of some preferred but not exclusive embodiments of a draining device according to the invention, made as an indication but not as a limitation with reference to the attached drawings.

### Brief description of the drawings

In such drawings:
- Figure 1 shows a perspective and partly sectioned view of a first embodiment of the draining device according to the invention, inserted through a patient's skin and in a non-operative condition;
- Figure 2 shows a perspective view of the draining device of figure 1 in an operative condition;
- Figure 3 shows a perspective and partly sectioned view of some details of a second embodiment of the draining device according to the invention, inserted through a patient's skin and in an operative condition;
- Figure 4 shows a perspective and enlarged view, partly in section, of a third embodiment of the draining device according to the invention, in a non-operative condition;
- Figure 5 shows a perspective and enlarged view, partly in section, of a fourth embodiment of the draining device according to the invention, during an expansion step of its interspaces;
- Figure 6 shows a perspective view of a fifth embodiment of the draining device according to the invention, inserted through a patient's skin and in an operative condition;
- Figure 7 shows a perspective and enlarged view of some details of a further embodiment of the draining device according to the invention.

### Detailed description of preferred embodiments

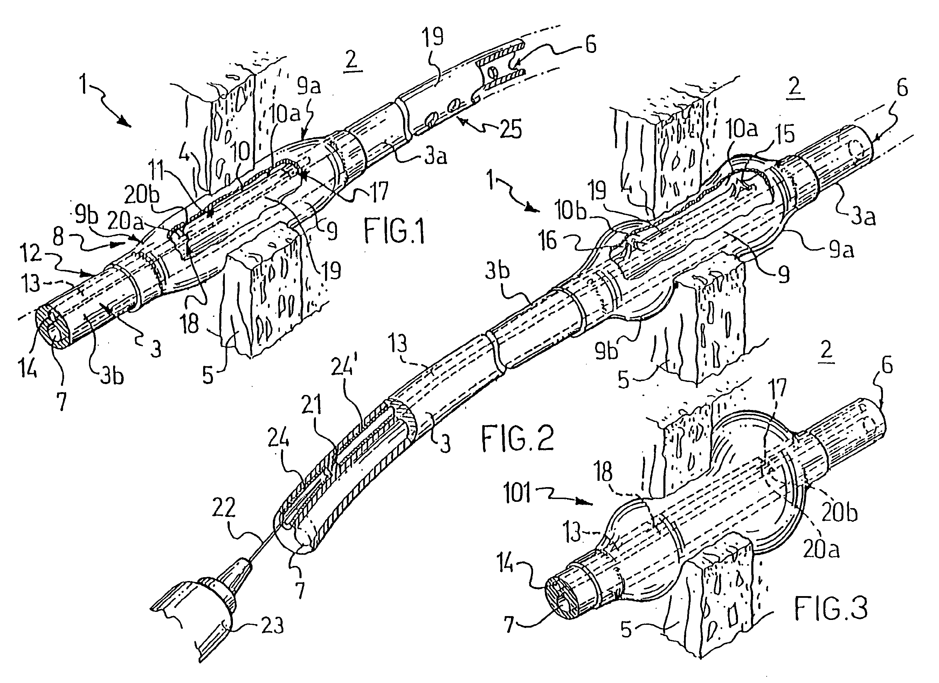

With reference to figures 1 and 2, a draining device adapted, in particular but not exclusively, for removing exudative material from an endocorporeal cavity 2 formed as a consequence of surgery on a patient, is generally indicated at 1.

The device 1 comprises a draining tube 3, intended to be housed - as it will be better shown hereafter - in an opening 4, made in a known way in the patient's skin 5, at a predetermined distance from the sutural margins of an operation wound, not shown, which tube is provided with opposite inlet and discharge openings 6, 7 of the exudative material collected in the cavity 2, facing the cavity 2 and, respectively, the environment external to the skin 5.

The discharge opening 7 is, in turn, in fluid communication with a container, conventional per se and not shown, for collecting and then disposing the exudative material drained from the cavity 2.

For the purposes of the invention, the draining tube 3 is made of a suitable biocompatible and sterilizable plastics material; preferably, the draining tube 3 is made of silicone rubber, natural rubber or another suitable elastomer.

The draining tube 3 of which, for simplicity, an intracutaneous portion 3a and an extracutaneous portion 3b are shown in the drawings, may have a circular cross-section or, more preferably, a substantially elliptical cross-section, so as to facilitate the cicatrization of the margins of the opening 4 after removal of the tube.

Advantageously, the intracutaneous portion 3a of the draining tube 3 is generally provided with a plurality of openings 25, radially formed through a wall 19 of the draining tube 3 near the inlet opening 6, and adapted to facilitate the removal of the exudative material from the cavity 2.

The device 1 is provided with fastening means 8 intended to fasten the draining tube 3 to the skin 5 of a patient, comprising a deformable sleeve 9, which coaxially and externally extends around the draining tube 3 along a portion thereof having a predetermined length.

The sleeve 9 is sealingly fastened - for example heat-sealed - to the draining tube 3, at end portions 9a, 9b of the latter, respectively intracutaneous and extracutaneous, so as to define an interspace 10 which is hermetically sealed and expandable under the action of a suitable sterile fluid.

The sleeve 9 is made by using a biocompatible and sterilizable plastic material, having a thickness ranging between 0.1 and 0.5 mm, having good characteristics of deformability and elasticity.

Preferably, the sleeve 9 is made of silicone rubber, rubber latex or any other plastics meeting the above requirements of biocompatibility, sterilizability and deformability.

In order to reduce the onset of decubitus phenomena at the sleeve 9, furthermore, the roughness of the outer surface of the latter may be properly increased, for example by means of the so-called conventional "texturing" processes.

Alternatively, the decubitus phenomena may be reduced by coating the sleeve 9 with a carbon-containing film, such as for example those marketed by Sorin Biomedica with the trade name of Carboflex^{™}.

For the purposes of the invention, the sleeve 9 extends externally around the draining tube 3 along a portion thereof having a predetermined length, previously determined according to the thickness of the skin 5 at the point of insertion of the tube itself.

Advantageously, the device 1 comprises a mark 11, for example a spot or a colored portion, formed at a predetermined position on the draining tube 3 and intended to facilitate - as it will be better apparent hereinafter - the correct positioning of the sleeve 9 astride the opening 4.

According to the invention, the interspace 10 may be expanded under the action of a suitable sterile fluid, preferably a sterile physiological solution, which is fed therein by means generally indicated at 12.

The means 12 for feeding the sterile fluid comprises a duct 13, substantially integral with the draining tube 3, having an inlet opening 14, accessible from the outside of the draining tube 3, and a pair of outlet openings 15, 16, selectively in fluid communication with end portions 10a, 10b, respectively intracutaneous and extracutaneous, of the interspace 10.

More particularly, the outlet openings 15, 16 are defined by opening respective check-valve means 17, 18, intended to prevent an accidental release of the sterile expansion fluid out of the end portions 10a, 10b of the interspace 10.

Advantageously, the check-valve means 17, 18, are essentially constituted by matching adjoining portions, elastically deformable, of the wall 19 of the draining tube 3, which are defined by a pair of through cross-notches 20a, 20b, radially formed in the wall 19 of the draining tube 3, and essentially perpendicular with one another.

Advantageously, the duct 13 for feeding the sterile fluid is sealingly closed by suitable means such as, for example, a self-sealing pierceable pad 21, fixed near the inlet opening 14, which may be pierced with a conventional needle 22 of a syringe 23, during the step of feeding the sterile fluid, to be afterwards closed again at the end of said step, thus preventing an outwardly directed leakage of the residual fluid that might be present in the feeding duct 13.

Alternatively, the feeding duct 13 may be sealingly closed by a removable plug, not shown, made of a suitable material and having a suitable shape.

Finally, the feeding duct 13 is provided with one, preferably two reinforcing tubular elements 24, 24', respectively mounted upstream and downstream of the pad 21.

The operation of the draining device 1 described hereinabove is as follows.

After having made - in a known way, for example with a special needle, conventional per se and not shown - an opening 4 through a patient's skin 5, the draining tube 3 is inserted through the same by carefully arranging the sleeve 9 symmetrically astride the skin 5.

Advantageously, the correct positioning of the sleeve 9 is facilitated by the mark 11, which delimits the extracutaneous portion 3b of the draining tube 3.

In addition, by arranging an intracutaneous portion 3a of suitable length, the mark 11 allows to carry out a simultaneous positioning of the inlet openings 6 and 25 in the cavity 2.

On the other hand, the discharge opening 7 of the draining tube 3 is connected - in a known way not shown - to a container adapted to collect and dispose the exudative material drained from the cavity 2.

Once the draining tube 3 has been positioned, the deformable sleeve 9 is expanded by gradually injecting a predetermined amount of a sterile physiological solution in the interspace 10 by means of the needle 22 of the syringe 23.

Thanks to the presence of the openings 15 and 16, the physiological solution is fed at the same time to the end portions 10a, 10b of the interspace 10, which is thus radially and symmetrically expanded with respect to the skin 5 (see figure 2).

The sleeve 9 will thus assume a "sandglass" configuration, adapted to firmly fasten the draining tube 3 to the skin 5 without having to make stitches.

During the step of injecting the sterile physiological solution, the valve means 17 and 18, constituted by the elastically deformable adjoining portions of the wall 19 of the draining tube 3, radially open outwards at the notches 20a, 20b under the effect of the pressure exerted by the expansion fluid, thus allowing a fluid communication between the feeding duct 13 and the interspace 10 through the openings 15, 16.

After having injected the physiological solution, said portions of the wall 19 match again with one another, so as to prevent the outflow of the physiological solution and to firmly keep the device in position. Additionally, the needle 22 may be extracted without leaks of fluid thanks to the presence of the pad 21 or of the removable plug, if present.

When the draining tube 3 is to be removed, for example at the end of the postoperative course, it is sufficient to repeat the operations described above in the reverse order, by inserting the needle 22 into the duct 13 and sucking the physiological expansion solution by means of the syringe 23. In this case, the suction causes a depression into the duct 13 adapted to cause a reverse opening of the valve means 17 and 18 constituted by the elastically deformable adjoining portions of the wall 19.

As soon as the sleeve 9 has returned to its initial configuration (figure 1), the draining tube 3 may be pulled out of the skin 5, which is then treated so as to prevent infections.

Advantageously, the possible use of a draining tube 3 with a substantially elliptical cross-section facilitates the healing and following cicatrization of the opening 4, so as to obtain an even more satisfactory aesthetic result.

Figures 3 - 7 show further embodiments of the draining device according to the invention, generally indicated respectively at 101, 201, 301 401 and 501.

In the following description, the elements of these draining devices structurally or functionally equivalent to those of device 1 will be referred to with the same reference numerals, and will not be further described.

In the device 101 shown in figure 3, the end portion 9a of the sleeve 9 has different elasticity characteristics and, more precisely, a greater expansion capability with respect to that of the remaining portion of the sleeve.

For the purposes of the invention, said characteristics of a greater expansion capability may be obtained both by properly reducing the longitudinal extension of the end portion 9a of the sleeve 9, or by manufacturing the latter with a different material having a greater degree of deformability.

In this way, the feeding of the sterile physiological solution will cause a differentiated expansion of the end portions 10a, 10b of the interspace 10, and the sleeve 9 will take the configuration shown in figure 3.

More particularly, the intracutaneous end portion 10a of the interspace 10 will expand transversely to the draining tube 3, with a prevailing radial development, so as to reduce to the minimum the volume taken by the draining device 101 in the cavity 2.

Advantageously, in addition, the overall size of the device 101 may be further reduced by suitably adjusting the size of the intracutaneous portion 3a of the draining tube 3.

This embodiment of the draining device may be preferably and advantageously applied where the skin 5 is especially thin and/or when the cavity 2 is close to important vascular-nervous structures, such as for example, the jugular vein, the carotid artery, and the phrenic nerve, present in the cervical region.

According to a further embodiment (not shown) of the device 101 shown in figure 3, both end portions 9a, 9b of the sleeve 9 may have a greater expansion capability with respect to a central portion of the sleeve 9 itself.

In this case, both end portions 10a, 10b of the interspace 10 will expand transversely to the draining tube 3 with a prevailing radial development, so as to reduce to the minimum the volume taken by the draining device 101 both in the cavity 2 and out of the skin 5.

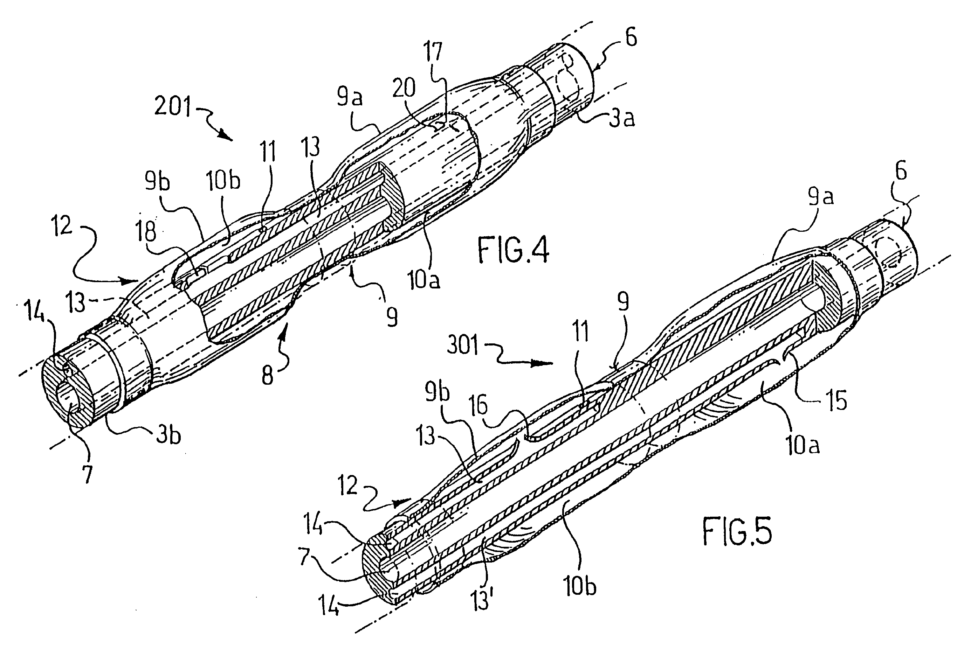

In the device 201 shown in figure 4, the end portions 9a, 9b of the sleeve 9 are structurally independent from each other and define respective expandable interspaces 10a, 10b, which are structurally independent as well.

Thanks to this feature, this embodiment also allows to prevent any accidental leakage of the expansion fluid between the above interspaces at the skin 5, thus keeping the device even more firmly fastened to the skin 5.

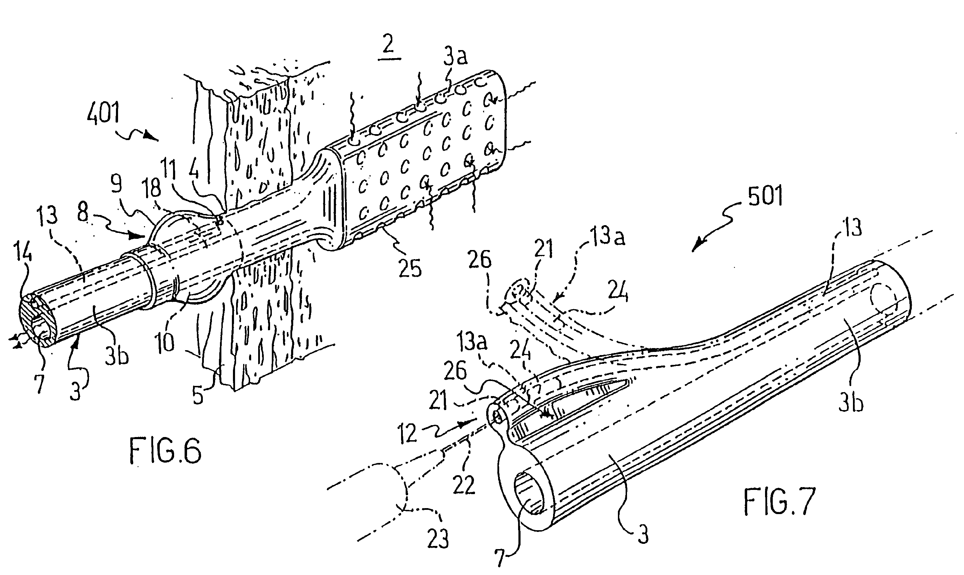

In the device 301 shown in figure 5, the feeding means 12 comprises two feeding ducts 13, 13' associated to the draining tube 3 at diametrically opposite sides thereof. In particular, each feeding duct 13, 13' is selectively in fluid communication with one of the expandable interspaces 10a, 10b.

In this way, the sterile fluid fed to a duct expands the interspace in fluid communication with the same, thus advantageously allowing an independent expansion of each expandable interspace 10a, 10b.

In the device 401 shown in figure 6, the intracutaneous portion 3a of the draining tube 3 has a flattened shape and is provided by openings 25 substantially along its entire length.

In this case, the sleeve 9 externally and coaxially extends around the draining tube 3 for a portion of predetermined length of the extracutaneous portion 3b only.

The injection of sterile physiological solution thus causes an expansion of the sleeve 9 only in the external part of the device 401, so as to prevent any accidental penetration of the tube into the cavity 2.

Clearly, this additional embodiment of the draining device of the invention may be used only when the cavity 2 is located in a region substantially without delicate internal organs, in which the skin has a suitable thickness.

In the device 501 shown in figure 7, the feeding duct 13 is only partly formed in the thickness of the wall 19 of the draining tube 3. In particular, the feeding duct 13 comprises an end portion 13a which is externally associated to the draining tube 3 by means of a resectable tongue 26.

Advantageously, said end portion 13a may be made structurally independent from the draining tube, thus facilitating the feeding of the sterile fluid; in particular, said embodiment allows to prevent accidental perforations of the draining tube 3 by the needle 22 while the fluid itself is being fed.

In addition, said end portion 13a allows to feed additional portions of the sterile fluid while using the device without removing the discharge opening 7 from the container used for collecting the drained exudative material.

The device 501 comprises a single reinforcing element 24 mounted in the feeding duct 13 downstream of the pad 18. In this way, the reinforcing element 24 prevents any accidental sliding of the pad 21 along the feeding duct 13 under the action of the needle 22 of the syringe 23 used to feed the sterile fluid.

## Claims

1. Device for draining exudative material from an endocorporeal cavity (2) of a patient, comprising:
a) a draining tube (3) having opposite inlet (6) and discharge (7) openings for the exudative material;
b) means (8) for fastening said draining tube (3) to the patient's skin (5), comprising a deformable sleeve (9), externally extending around the draining tube (3) to which the same is sealingly fixed, and with which the sleeve defines an expandable interspace (10);
c) means (12) for feeding a sterile expansion fluid to at least one end portion (10a, 10b) of said expandable interspace (10), said means (12) including at least one duct (13, 13') at least partly integrally formed in said draining tube (3), having an inlet opening (14) accessible from the outside of the draining tube (3) and at least one outlet opening (16) selectively in fluid communication with said at least one end portion (10a, 10b) of the interspace (10),
wherein said at least one outlet opening (16) is defined by at least one through notch (20) radially formed in a wall (19) of the draining tube (3) between said at least one duct (13, 13') and said at least one end portion (10a, 10b) of the interspace (10) and in that said at least one through notch (20) defines a two-way check-valve means (18) adapted to prevent an accidental release of the sterile fluid from said at least one end portion (10a, 10b) of the interspace (10) when the draining tube (3) is fastened to the patient's skin (5) and to inflate or deflate said deformable sleeve (9) by feeding or sucking said sterile fluid to and from said at least one end portion (10a, 10b) of said expandable interspace (10).

2. Draining device according to claim 1, **characterized in that** said check-valve means (18) is essentially constituted by elastically deformable adjoining portions of the wall (19) of the draining tube (3), said portions being defined by said at least one through notch (20).

3. Draining device according to claim 1, **characterized in that** said deformable sleeve (9) comprises two portions (9a, 9b) structurally independent from one another, and defining respective expandable interspaces (10a, 10b) with said draining tube (3).

4. Draining device according to claim 1 or 3, **characterized in that** said means (12) for feeding the sterile fluid comprises two feeding ducts (13, 13') at least partly integrally formed in said draining tube (3) at diametrically opposite sides thereof, each of said feeding ducts (13, 13') being selectively in fluid communication with one of said expandable interspaces (10a, 10b).

5. Draining device according to claim 1, **characterized in that** said deformable sleeve (9) coaxially extends around the draining tube (3) along a portion thereof having a predetermined length.

6. Draining device according to claim 1, **characterized in that** said at least one duct (13) for feeding the sterile fluid is provided near said inlet opening (14) with at least one reinforcing tubular element (24, 24').

7. Draining device according to claim 6, **characterized in that** said at least one reinforcing tubular element (24, 24') is made of metal or plastics.

8. Draining device according to claims 1 or 4, **characterized in that** it further comprises means for sealingly closing said at least one feeding duct (13, 13').

9. Draining device according to claim 8, **characterized in that** said means comprises a self-sealing pierceable pad fastened in said at least one feeding duct (13, 13') near said inlet opening (14).

10. Draining device according to claim 9, **characterized in that** said pad (21) is mounted in said at least one feeding duct (13, 13') upstream of said at least one reinforcing element (24).

11. Draining device according to claim 9, **characterized in that** said pad (21) is mounted in said at least one feeding duct (13, 13') between two reinforcing elements (24, 24').

12. Draining device according to claim 8, **characterized in that** said means comprises a suitably shaped plug, removably fitted in the inlet opening (14) of said at least one feeding duct (13, 13').

13. Draining device according to claim 1, **characterized in that** said at least one feeding duct (13, 13') comprises an end portion externally fastened to the draining tube (3) by means of a resectable tongue (26).

14. Draining device according to claim 1, **characterized in that** said sleeve (9) comprises at least one end portion (9a, 9b) having a different elasticity.

15. Draining device according to claim 1, **characterized in that** said draining tube (3) comprises a flattened intracutaneous portion (3a) provided with a plurality of openings (25) for sucking the exudative material.

16. Draining device according to anyone of the preceding claims, **characterized in that** said draining tube (3) has a substantially elliptical cross-section.

17. Draining device according to anyone of the preceding claims, **characterized in that** it further comprises a mark (11) formed at a predetermined position on said draining tube (3), for indicating the correct positioning of the device through the skin (6) of the patient.

## Patentansprüche

1. Vorrichtung zum Ableiten von abgesondertem Material aus einer Körperhöhle (2) eines Patienten, mit:
a) einem Drainageschlauch (3), der gegenüberliegende Einlassöffnungen (6) und Auslassöffnungen (7) für das abgesonderte Material aufweist;
b) Mitteln (8) zum Befestigen des Drainageschlauchs (3) an der Haut (5) des Patienten, die eine verformbare Hülse (9) aufweisen, die sich außen um den Drainageschlauch (3) erstreckt, an weichem sie abdichtend befestigt ist, und mit dem die Hülse einen erweiterbaren Zwischenraum (10) definiert;
c) Mitteln (12) zum Zuführen eines sterilen Expansionsfluids zu mindestens einem Endabschnitt (10a, 10b) des erweiterbaren Zwischenraums (10), wobei die Mittel (12) mindestens eine Röhre (13, 13') umfassen, die mindestens teilweise integral in dem Drainageschlauch (3) ausgebildet ist, mit einer Einlassöffnung (14), die von außerhalb des Drainageschlauchs (3) zugänglich Ist, und mit mindestens einer Auslassöffnung (16), die wahlweise in Fluidverbindung mit dem mindestens einen Endabschnitt (10a, 10b) des Zwischenraumes (10) stehen,
wobei die mindestens eine Auslassöffnung (16) durch mindestens eine Durchgangsöffnung (20), die radial in einer Wand (19) des Drainageschlauchs (3) zwischen der mindestens einen Röhre (13, 13') und dem mindestens einen Endabschnitt (10a, 10b) des Zwischenraums (10) ausgebildet ist, definiert ist, und wobei die mindestens eine Durchgangsöffnung (20) eine Zweiwege-Klappenventilvorrichtung (18) definiert, die geeignet ist, ein unbeabsichtigtes Entlassen des sterilen Fluids aus dem mindestens einen Endabschnitt (10a, 10b) des Zwischenraums (10) zu verhindern, wenn der Drainageschlauch (3) an der Haut (5) des Patienten befestigt ist, und die geeignet ist, die verformbare Hülse (9) durch Zuführen oder Saugen des sterilen Fluids in den oder aus dem mindestens einen Endabschnitt (10a, 10b) des erweiterbaren Zwischenraumes (10) zu füllen oder zu entleeren.

2. Drainagevorrichtung nach Anspruch 1, **dadurch gekennzeichnet, dass** die Klappenventilvorrichtung (18) im Wesentlichen durch elastisch verformbare benachbarte Abschnitte der Wand (19) des Drainageschlauchs (3) ausgebildet Ist, wobei die Abschnitte durch die mindestens eine Durchgangsöffnung (20) definiert sind.

3. Drainagevorrichtung nach Anspruch 1, **dadurch gekennzeichnet, dass** die verformbare Hülse (9) zwei Abschnitte (9a, 9b) aufweist, die strukturell unabhängig voneinander sind, und die entsprechend erweiterbare Zwischenräume (10a, 10b) mit dem Drainageschlauch (3) definieren.

4. Drainagevorrichtung nach Anspruch 1 oder 3, **dadurch gekennzeichnet, dass** die Mittel (12) zum Zuführen des sterilen Fluids zwei Zuführröhren (13, 13') aufweisen, die zumindest teilweise integral in dem Drainageschlauch (3) an diametral gegenüberliegenden Seiten davon ausgebildet sind, wobei jede der Zuführröhren (13, 13') wahlweise in Fluidverbindung mit einem der erweiterbaren Zwischenräume (10a, 10b) stet.

5. Drainagevorrichtung nach Anspruch 1, **dadurch gekennzeichnet, dass** sich die verformbare Hülse (9) koaxial um den Drainageschlauch (3) entlang eines Abschnitts davon erstreckt, der eine vorbestimmte Länge aufweist.

6. Drainagevorrichtung nach Anspruch 1, **dadurch gekennzeichnet, dass** die mindestens eine Röhre (13) zum Zuführen des sterilen Fluids in der Nähe der Einlassöffnung (14) mit mindestens einem verstärkenden Schlauchelement (24, 24') vorgesehen ist.

7. Drainagevorrichtung nach Anspruch 6, **dadurch gekennzeichnet, dass** das mindestens eine Schlauchelement (24, 24') aus Metall oder Kunststoff hergestellt ist.

8. Drainagevorrichtung nach Anspruch 1 oder 4, **dadurch gekennzeichnet, dass** sie des Weiteren Mittel zum abdichtenden Schließen der mindestens einen Zuführröhre (13, 13') aufweist.

9. Drainagevorrichtung nach Anspruch 8, **dadurch gekennzeichnet, dass** die Mittel eine selbstdichtende durchdringbare Zwischenlage aufweisen, die in der mindestens einen Zuführröhre (13, 13') in der Nähe der Einlassöffnung (14) angebracht ist.

10. Drainagevorrichtung nach Anspruch 9, **dadurch gekennzeichnet, dass** die Zwischenlage (21) in der mindestens einen Zuführröhre (13, 13') stromaufwärts von dem mindestens einen Verstärkungselement (24) eingebaut ist.

11. Drainagevorrichtung nach Anspruch 9, **dadurch gekennzeichnet, dass** die Zwischenlage (21) in der mindestens einen Zuführröhre (13, 13') zwischen zwei Verstärkungselementen (24, 24') eingebaut ist.

12. Drainagevorrichtung nach Anspruch 8, **dadurch gekennzeichnet, dass** die Mittel einen in geeigneter Weise geformten Stöpsel aufweisen, der entfembar in die Einlassöffnung (14) der mindestens einen Zuführröhre (13, 13') eingepasst ist.

13. Drainagevorrichtung nach Anspruch 1, **dadurch gekennzeichnet, dass** die mindestens eine Zuführröhre (13, 13') einen Endabschnitt aufweist, der außen an den Drainageschlauch (3) mit Hilfe einer herausschneidbaren Zunge (26) befestigt ist.

14. Drainagevorrichtung nach Anspruch 1, **dadurch gekennzeichnet, dass** die Hülse (9) mindestens einen Endabschnitt (9a, 9b) mit einer unterschiedlichen Elastizität aufweist.

15. Drainagevorrichtung nach Anspruch 1, **dadurch gekennzeichnet, dass** der Drainageschlauch (3) einen abgeflachten intrakutanen Abschnitt (3a) aufweist, der mit einer Vielzahl von Öffnungen (25) zum Heraussaugen des abgesonderten Materials ausgestattet ist.

16. Drainagevorrichtung nach einem der vorhergehenden Ansprüche, **dadurch gekennzeichnet, dass** der Drainageschlauch (3) einen im Wesentlichen elliptischen Querschnitt aufweist.

17. Drainagevorrichtung nach einem der vorhergehenden Ansprüche, **dadurch gekennzeichnet, dass** sie des Weiteren eine Markierung (11) aufweist, die an einer vorbestimmten Position auf dem Drainageschlauch (3) zum Angeben der korrekten Positionierung der Vorrichtung durch die Haut (6) des Patienten ausgebildet ist.

## Revendications

1. Dispositif de drainage d'un exsudat d'une cavité endocorporelle (2) d'un patient, qui comprend :
a) un tube de drainage (3) ayant des orifices d'entrée (6) et de décharge (7) opposés pour l'exsudat ;
b) des moyens (8) de fixation dudit tube de drainage (3) à la peau du patient (5), comprenant un manchon déformable (9), s'étendant à l'extérieur autour du tube de drainage (3) auxquels celui-ci est fixé de manière étanche, et avec lesquels le manchon définit un espace expansible (10) ;
c) des moyens (12) d'alimentation d'un fluide de dilatation stérile à au moins une partie d'extrémité (10a, 10b) dudit espace expansible (10), lesdits moyens (12) comprenant au moins un canal (13, 13'), au moins partiellement intégralement formé dans ledit tube de drainage (3), ayant un orifice d'entrée (14) accessible depuis l'extérieur du tube de dragage (3) et au moins un orifice de sortie (16) sélectivement en communication fluidique avec ladite au moins une partie d'extrémité (10a, 10b) de l'espace (10),
dans lequel ledit au moins un orifice de sortie (16) est défini par au moins une encoche débouchante (20), formée radialement dans une paroi (19) du tube de drainage (3) entre ledit au moins un conduit (13, 13') et ladite au moins une partie d'extrémité (10a, 10b) de l'espace (10) et en ce que ladite au moins une encoche débouchante (20) définit des moyens de clapet double (18) adaptés pour empêcher une libération accidentelle du fluide stérile de ladite au moins une partie d'extrémité (10a, 10b) de l'espace (10) lorsque le tube de drainage (3) est attaché à la peau du patient (5) et pour gonfler ou dégonfler ledit manchon déformable (9) en alimentant ou en aspirant ledit fluide stérile à ladite et de ladite au moins une partie d'extrémité (10a, 10b) dudit espace expansible (10).

2. Dispositif de drainage selon la revendication 1, **caractérisé en ce que** lesdits moyens de clapet (18) sont essentiellement constitués par des parties jointes élastiquement déformables de la paroi (19) du tube de drainage (3), lesdites parties étant définies par ladite au moins une encoche débouchante (20).

3. Dispositif de drainage selon la revendication 1, **caractérisé en ce que** ledit manchon déformable (9) comprend deux parties (9a, 9b) structurellement indépendantes l'une de l'autre, et définissant des espaces respectifs expansibles (10a, 10b) avec ledit tube de drainage (3).

4. Dispositif de drainage selon la revendication 1 ou 3, **caractérisé en ce que** lesdits moyens (12) d'alimentation du fluide stérile comprennent deux conduits d'alimentation (13, 13') au moins partiellement intégralement formés dans ledit tube de drainage (3) à des côtés diamétralement opposés de celui-ci, chacun desdits conduits d'alimentation (13, 13') étant sélectivement en communication fluidique avec un desdits espaces expansibles (10a, 10b).

5. Dispositif de drainage selon la revendication 1, **caractérisé en ce que** ledit manchon déformable (9) s'étend de manière coaxiale autour du tube de drainage (3) le long d'une partie de celui-ci ayant une longueur prédéterminée.

6. Dispositif de drainage selon la revendication 1, **caractérisé en ce que** ledit au moins un conduit (13) d'alimentation du fluide stérile est doté à proximité dudit orifice d'entrée (14) d'au moins un élément tubulaire de renforcement (24, 24').

7. Dispositif de drainage selon la revendication 6, **caractérisé en ce que** ledit au moins un élément tubulaire de renforcement (24, 24') est formé par du métal ou du plastique.

8. Dispositif de drainage selon les revendications 1 ou 4, **caractérisé en ce qu'**il comprend en outre des moyens de fermeture étanche dudit au moins un conduit d'alimentation (13, 13').

9. Dispositif de drainage selon la revendication 8, **caractérisé en ce que** lesdits moyens comprennent un coussin pouvant être percé à fermeture automatique fixé dans ledit au moins un conduit d'alimentation (13, 13') à proximité dudit orifice d'entrée (14).

10. Dispositif de drainage selon la revendication 9, **caractérisé en ce que** ledit coussin (21) est monté dans ledit au moins un conduit d'alimentation (13, 13') en amont dudit au moins un élément de renforcement (24).

11. Dispositif de drainage selon la revendication 9, **caractérisé en ce que** ledit coussin (21) est monté dans ledit au moins un conduit d'alimentation (13, 13') entre deux éléments de renforcement (24, 24'),

12. Dispositif de drainage selon la revendication 8, **caractérisé en ce que** lesdits moyens comprennent un bouchon ayant une forme adaptée, ajusté de manière amovible dans l'orifice d'entrée (14) dudit au moins un conduit d'alimentation (13, 13').

13. Dispositif de drainage selon la revendication 1, **caractérisé en ce que** ledit au moins un conduit d'alimentation (13, 13') comprend une partie d'extrémité attachée à l'extérieur du tube de drainage (3) par les moyens d'une languette résécable (26).

14. Dispositif de drainage selon la revendication 1, **caractérisé en ce que** ledit manchon (9) comprend au moins une partie d'extrémité (9a, 9b) ayant une élasticité différente.

15. Dispositif de drainage selon la revendication 1, **caractérisé en ce que** ledit tube de drainage (3) comprend une partie intracutanée aplatie (3a) dotée d'une pluralité d'ouvertures (25) pour aspirer l'exsudat.

16. Dispositif de drainage selon l'une quelconque des revendications précédentes, **caractérisé en ce que** ledit tube de drainage (3) a une section transversale sensiblement elliptique.

17. Dispositif de drainage selon l'une quelconque des revendications précédentes, **caractérisé en ce qu'**il comprend en outre un repère (11) formé à une position prédéterminée sur ledit tube de drainage (3), destiné à indiquer le positionnement correct du dispositif dans la peau (6) du patient.
